# EUROPEAN PATENT APPLICATION

(11) **EP 4 113 526 A1**
(43) Date of publication of application: **04.01.2023**
(21) Application number: 20928192.2
(22) Date of filing: 31.03.2020
(51) Int. Cl.: G16H 20/10

(54) **INFORMATION PROVISION DEVICE, INFORMATION MANAGEMENT DEVICE, USER TERMINAL, ACQUISITION METHOD, USER INTERFACE, DATABASE, INFORMATION PROVISION METHOD, AND CONTROL METHOD**

(71) Applicant: Nippon Telegraph And Telephone Corporation, Chiyoda-ku Tokyo 100-8116 (JP)
(72) Inventor: MOGAKI Takefumi, Musashino-shi, Tokyo 180-8585 (JP); NAGATA Kengo, Musashino-shi, Tokyo 180-8585 (JP); NAKATSUJI Rena, Musashino-shi, Tokyo 180-8585 (JP); NAGAI Yosuke, Musashino-shi, Tokyo 180-8585 (JP)
(74) Representative: Brevalex
(86) International application number: PCT/JP2020/014947
(87) International publication number: WO 2021/199352

(57) **Abstract**

An aspect of the present invention provides an information providing apparatus including: a communication unit that communicates with another device via a network; and a control unit that, based on information indicating a medicine requiring caution in use for a user, the information being obtained based on information relating to a gene of the user, and information indicating a medicine prescribed for the user, determines a medicine requiring caution in use for the user, the medicine requiring caution in use being of the medicine prescribed for the user, and provides information indicating a result of the determination to the other device.

## Description

### TECHNICAL FIELD

The present invention relates to an information providing apparatus, an information managing apparatus, a user terminal, an acquiring method, a user interface, a database, an information providing method and a control method.

### BACKGROUND ART

Conventionally, when medicine is provided to a patient, a pharmacist belonging to a dispensing facility dispenses the medicine to the patient according to a prescription issued by a doctor. For enhancement of convenience of dispensing according to a prescription, applications relating medicine have been provided. In some of such applications, medicine notebooks are electrically implemented (for example, Non-Patent Literature 1). Use of such application enables a user to easily manage information on medicines prescribed for himself/herself. Also, a pharmacist can more properly dispense medicine based on information relating to a medicine notebook recorded by the application.

### Citation List

### Non-Patent Literature

Non-Patent Literature 1: "Medicine Notebook", Japan Pharmaceutical Association, [online], [searched on February 10, 2020], Internet<URL http://www-eokusuri.nichiyaku.or.jp/index.html>

### SUMMARY OF THE INVENTION

### Technical Problem

However, even a same medicine varies among different individuals in, e.g., effectiveness, and easiness of occurrence of side effects of the medicine. Conventionally, it has been difficult to perform dispensing according to such individual differences.

In view of the above circumstances, an object of the present invention is to provide a technique that enables more proper dispensing according to individual difference relating to medicine.

### Means for Solving the Problem

An aspect of the present invention provides an information providing apparatus including: a communication unit that communicates with another device via a network; and a control unit that, based on information indicating a medicine requiring caution in use for a user, the information being obtained based on information relating to a gene of the user, and information indicating a medicine prescribed for the user, determines a medicine requiring caution in use for the user, the medicine requiring caution in use being of the medicine prescribed for the user, and provides information indicating a result of the determination to the other device.

An aspect of the present invention provides an information managing apparatus including: a communication unit that communicates with another device via a network; a storage unit that stores user gene information that is information relating to a gene of a user and caution medicine information indicating a medicine requiring caution in use for the user, the caution medicine information being obtained based on the user gene information; and a control unit that provides the caution medicine information to the other device.

An aspect of the present invention provides a user terminal including: a communication unit that communicates with another device via a network; an output unit that outputs information to a user; and a control unit that acquires information relating to a medicine requiring caution in use for the user, which is of the medicine prescribed for the user, and outputs the information relating to a medicine requiring caution in use to the user via the output unit.

An aspect of the present invention provides an acquiring method including in order to estimate an interaction between a plurality of medicines used by a user: a step of acquiring the medicines used by the user, an effect relating to a body of the user, the effect being exerted after use of the medicines by the user, and a time; and a step of performing at least one of processing for transmitting the acquired medicines, effect and time to another device and processing for storing the acquired medicines, effect and time in a storage device.

An aspect of the present invention provides a user interface including: a medicine input field that receives an input of information indicating a medicine used by the user; an effect input field that receives an input of information indicating an effect relating to a body of the user, the effect being exerted after use of the medicine by the user; and a transmission determination field that receives an instruction for transmission of the information input by the user.

An aspect of the present invention provides a database including: a storage unit that, in order to estimate an interaction between a plurality of medicines used by a user, stores the medicines used by the user, an effect relating to a body of the user, the effect being exerted after use of the medicines by the user, and a time in association with one another.

An aspect of the present invention provides an information providing method including: a step of, based on information indicating a pharmaceutical formulation requiring caution in use/administration for a user, the information being obtained based on information relating to a gene of the user, and information indicating a pharmaceutical formulation prescribed for the user, determining a pharmaceutical formulation requiring caution in use/administration for the user, the pharmaceutical formulation requiring caution in use/administration being of the pharmaceutical formulation prescribed for the user; and a step of providing information indicating a result of the determination to another device.

An aspect of the present invention provides a control method including: a step of acquiring information relating to a pharmaceutical formulation requiring caution in use/administration for a user, which is of the pharmaceutical formulation prescribed for the user; and a step of outputting the information relating to a pharmaceutical formulation requiring caution in use/administration to the user.

### Effects of the Invention

The present invention enables more proper dispensing according to individual differences relating to medicine.

### BRIEF DESCRIPTION OF DRAWINGS

[Fig. 1] Fig. 1 is a schematic block diagram illustrating a system configuration of an information provision system 100 of the present invention.
[Fig. 2] Fig. 2 is a schematic diagram illustrating an example configuration of a medicine information database 10.
[Fig. 3] Fig. 3 is a diagram illustrating a specific example of medicine information.
[Fig. 4] Fig. 4 is a schematic diagram illustrating an example configuration of a user gene information management device 20.
[Fig. 5] Fig. 5 is a diagram illustrating a specific example of caution medicine information.
[Fig. 6] Fig. 6 is a diagram illustrating a specific example of gene-related information.
[Fig. 7] Fig. 7 is a schematic diagram illustrating an example configuration of an information provision server 30.
[Fig. 8] Fig. 8 is a diagram illustrating a specific example of feedback information.
[Fig. 9] Fig. 9 is a schematic diagram illustrating an example configuration of a user terminal 40.
[Fig. 10] Fig. 10 is a diagram illustrating an example of an output mode of prescription information.
[Fig. 11] Fig. 11 is a diagram illustrating an example of an output mode in which gene-related information is displayed.
[Fig. 12] Fig. 12 is a diagram illustrating an example of an output mode in which gene-related information is displayed.
[Fig. 13] Fig. 13 is a diagram illustrating an example of an output mode of prescription information.
[Fig. 14] Fig. 14 is a diagram illustrating an example of an output mode in which gene-related information is displayed.
[Fig. 15] Fig. 15 is a diagram illustrating an example of an output mode of prescription information.
[Fig. 16] Fig. 16 is a diagram illustrating an example of an output mode in which gene-related information is displayed.
[Fig. 17] Fig. 17 is a diagram illustrating an example screen of a use reminder.
[Fig. 18] Fig. 18 is a diagram illustrating a specific example of a questionnaire screen.
[Fig. 19] Fig. 19 is a sequence chart illustrating a specific example of a flow of operation of the information provision system 100.
[Fig. 20] Fig. 20 is a sequence chart illustrating a specific example of a flow of operation of the information provision system 100.
[Fig. 21] Fig. 21 is a sequence chart illustrating a specific example of a flow of operation of the information provision system 100.
[Fig. 22] Fig. 22 is a schematic block diagram illustrating a system configuration of an alteration of the information provision system 100 of the present invention.
[Fig. 23] Fig. 23 is a sequence chart illustrating a specific example of a flow of operation of the alteration of the information provision system 100.

### DESCRIPTION OF EMBODIMENTS

First, key points of the present embodiment will be described. It has been known that there is a correlation between the aforementioned individual differences relating to medicine, that is, effectiveness of a predetermined medicine or side effects exhibited due to the predetermined medicine, and gene polymorphisms of human beings. In the present embodiment, based on this correlation, proper dispensing according to individual differences relating to medicine is performed by using a gene polymorphism of a user.

An example specific configuration of the present invention will be described with reference to the drawings.

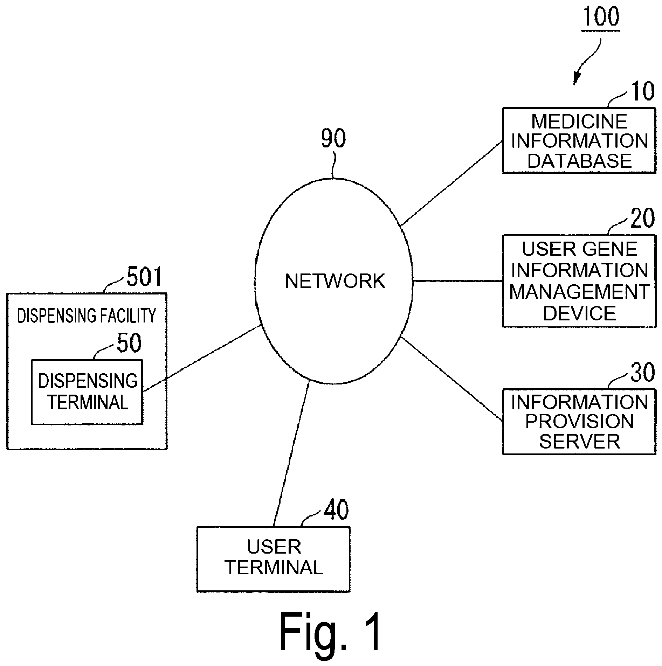

Fig. 1 is a schematic block diagram illustrating a system configuration of an information provision system 100 of the present invention. The information provision system 100 is a system for providing information relating to medicine with individual differences in, e.g., effectiveness of the medicine and/or easiness of occurrence of side effects taken into consideration (hereinafter referred to as "medicine-related information") to a user or a person concerned with medicine. A person concerned with medicine is a person who determines a type and quantity of medicine to be used for a user. An act of medicine being used for a user may be administration of the medicine to the user, may be the user's taking of the medicine, patching on the body of the user, inunction of the medicine to the body of the user, or injection of the medicine into the user. The act may be any act of providing the medicine to the body of the user. A medicine to be used may be a preparation to be taken, a medicine to be applied or a medicine to be injected and thus may be any type of medicine to be provided to a user. The person concerned with medicine is, for example, a doctor or a staff member in a dispensing facility (for example, a pharmacist). Effectiveness of medicine indicates a degree of change in condition of a user due to provision of a predetermined unit quantity of a predetermined medicine to the user. Effectiveness of medicine may be defined as information indicating a degree of difficulty of effects emerging even with a regular dose. Where a speed of decomposition of medicine is high because of a metabolic enzyme produced by a gene polymorphism, the administered medicine is less effective. Medicine-related information may indicate a specific genetic name or product name of medicine, may be information indicating a collection of medicines having a same function (e.g., a plurality of product names of medicines containing same components or product names of generic medicines) or may indicate names of components.

The information provision system 100 includes a medicine information database 10, a user gene information management device (managing apparatus) 20, an information provision server 30, a user terminal 40 and a dispensing terminal 50. The medicine information database 10, the user gene information management device 20, the information provision server 30, the user terminal 40 and the dispensing terminal 50 are communicably connected via a network 90. The network 90 may be a network using wireless communication or a network using wired communication. The network 90 may be formed by a combination of a plurality of networks.

The medicine information database 10 stores information indicating a relationship between each medicine and genes (hereinafter referred to as "medicine information"). The user gene information management device 20 stores information relating to genes for each user (hereinafter referred to as "user gene information"). The user gene information management device 20 stores medicine-related information for each user. The medicine-related information is obtained based on the medicine information and the user gene information. The medicine-related information includes caution medicine information and gene-related information. The caution medicine information is information indicating, for each user, medicine requiring caution for the user. For example, the caution medicine information may include information relating to medicine requiring caution for, e.g., side effects in excess of a predetermined level in use, may include information relating to medicine that is likely to exert only an effect that is below a predetermined level even if the medicine is used and may include information relating to a side effect that may occur when the medicine is used. The gene-related information further includes information relating to genes that cause the medicines to require caution in addition to the caution medicine information. A criterion for caution may properly be set in the information provision system 100 according to, e.g., a degree of caution, the degree being determined by a relationship between a user and medicine and/or social situations. For example, a criterion for caution may be determined according to any of various criterions such as a threshold value for allergic reaction and a ratio of occurrence of a predetermined degree of drowsiness in users who used the medicine.

The user gene information management device 20 normally provides caution medicine information to the information provision server 30. Based on the caution medicine information, the information provision server 30 provides information relating to medicine requiring caution for a relevant user to the user terminal 40 or the dispensing terminal 50. For example, if a prescription given to a user includes medicine requiring caution for the user, an alert may be issued. Such caution medicine information includes no information relating to an individual gene. Therefore, it is possible to provide information according to individual differences relating to medicine while keeping individual gene information secret.

Furthermore, if a predetermined condition indicating obtainment of a user's permission (hereinafter referred to as "permission condition") is met, the user gene information management device 20 provides a part or an entirety of gene-related information to another device (for example, the user terminal 40 or the dispensing terminal 50). The gene-related information includes information relating to the user's genes as information that is more detail than the caution medicine information. The gene-related information to be provided is partly or entirely permitted by the user. For example, the permission may be provided by the user in such a manner that only gene-related information relating to a particular disease is provided. For example, the permission may be provided by the user in such a manner that only gene-related information relating to a particular medicine is provided. Such gene-related information is provided only when the permission condition is met, enabling the gene-related information to be treated as secret because of the user's private information. Also, if the permission condition is met, gene-related information is provided to another device, enabling a pharmacist to acquire the gene-related information and perform more proper dispensing. Details of the information provision system 100 will be described below.

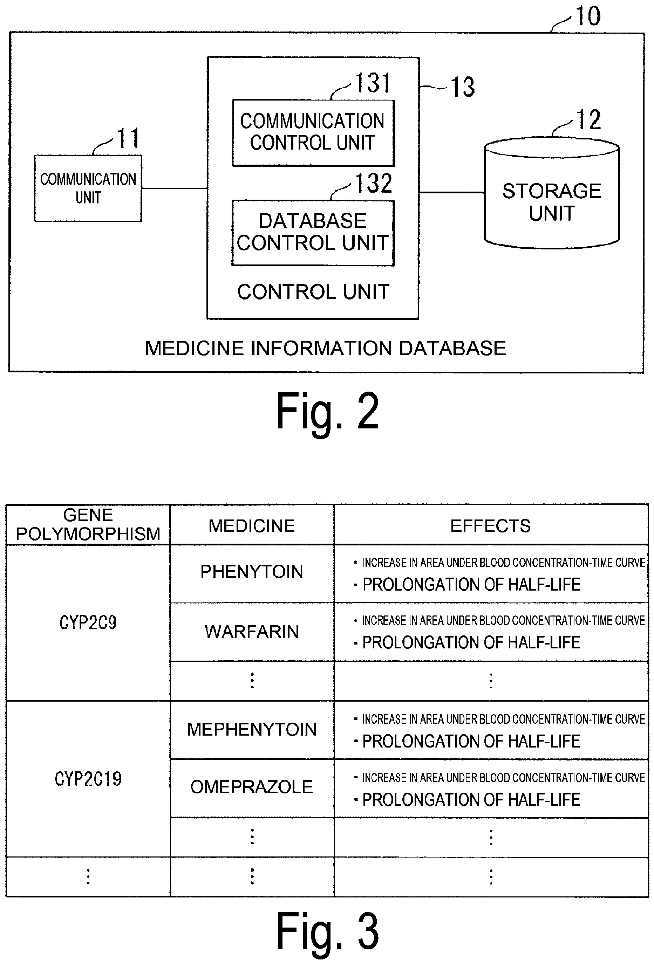

Fig. 2 is a schematic diagram illustrating an example configuration of the medicine information database 10. The medicine information database 10 is made up of an information processing device such as a personal computer or a server device. The medicine information database 10 includes a communication unit 11, a storage unit 12 and a control unit 13.

The communication unit 11 is a communication device. The communication unit 11 may be configured as, for example, a network interface. The communication unit 11 performs data communication with another device via the network 90 according to control by the control unit 13. The communication unit 11 may be a device that performs wireless communication or may be a device that performs wired communication.

The storage unit 12 is made up of a storage device such as a magnetic hard disk device or a semiconductor storage device. The storage unit 12 stores data to be used by the control unit 13. The storage unit 12 stores, for example, medicine information. Fig. 3 is a diagram illustrating a specific example of the medicine information. Medicine information includes certain gene information and information relating to medicines that can affect metabolism if such gene information is included. Gene information associated with medicines and effects may be information indicating gene polymorphisms, information indicating genotypes (sets of genes) or information indicating expressed genes. Medicine information includes, for example, information relating to medicines that can affect metabolism if a mutation such as a defect occurs in a certain gene. A gene polymorphism is an individual difference in a DNA sequence forming a gene and affects metabolism of medicine because of the individual difference.

For example, a medicine called phenytoin is sometimes used for a brain-injured patient for the purpose of preventing a symptomatic epileptic seizure. An overly high blood concentration of phenytoin causes, e.g., dizziness or coma as a side effect. Therefore, it is necessary to prevent an overly high blood concentration of phenytoin. On the other hand, CYP2C9, which is a molecule molecular species of CYP, is involved in metabolism of phenytoin, and phenytoin may fail to be properly metabolized in an individual with a mutation occurring in CYP2C9. In other words, in an individual with a mutation occurring in CYP2C9, an increase in area under a blood concentration-time curve or prolongation of a half-life is highly likely to be recognized. Therefore, for such individual (user), caution is necessary in prescription of phenytoin. Medicine information includes information relating to medicine metabolism occurring according to a gene polymorphism in this way. Such medicine information can be obtained by, for example, based on, e.g., articles relating to medicine and genes and/or test results. The medicine information is properly updated according to, e.g., new articles and/or new test results.

The control unit 13 is made up of a processor such as a CPU (central processing unit) and a memory. The control unit 13 functions a communication control unit 131 and a database control unit 132 by the processor executing a program. Note that all or some of the functions of the control unit 13 may be implemented by using hardware such as an ASIC (application-specific integrated circuit), a PLD (programmable logic device) or an FPGA (field-programmable gate array). The above program may be recorded on a computer-readable recording medium. The computer-readable recording medium is, for example, a portable medium such as a flexible disk, a magneto-optical disk, a ROM, a CD-ROM or a semiconductor storage device (for example, an SSD (solid-state drive)) or a storage device, such as a hard disk or a semiconductor storage device, incorporated in a computer system. The above program may be transmitted via a telecommunication channel.

The communication control unit 131 controls communication with another device via the communication unit 11. The database control unit 132 updates the medicine information stored in the storage unit 12. For example, where new medicine information is obtained from the other device, the database control unit 132 updates the medicine information based on the obtained information. If there is a request for provision of medicine information from the other device, the database control unit 132 provides a part or an entirety of the medicine information stored in the storage unit 12. The database control unit 132 may be configured in such a manner as to provide a part or an entirety of the medicine information stored in the storage unit 12 to a specifically certified device only.

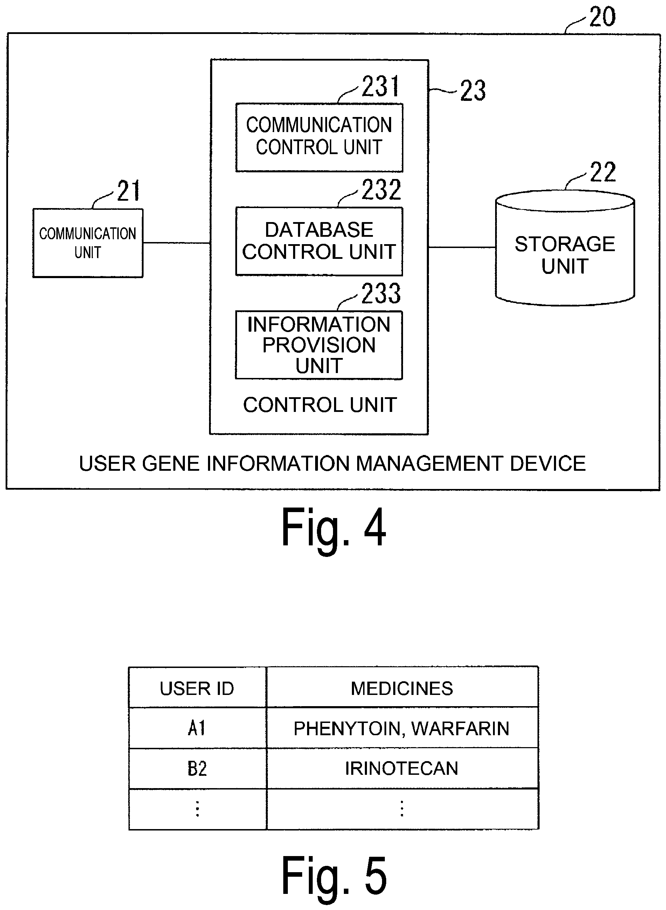

Fig. 4 is a schematic diagram illustrating an example configuration of the user gene information management device 20. The user gene information management device 20 is made up of an information processing device such as a personal computer or a server device. The user gene information management device 20 is desirably a device managed by an organization asked for analysis of gene information by a user or an organization allowed to manage gene information. The user gene information management device 20 includes a communication unit 21, a storage unit 22 and a control unit 23.

The communication unit 21 is a communication device. The communication unit 21 may be configured as, for example, a network interface. The communication unit 21 performs data communication with another device via the network 90 according to control by the control unit 23. The communication unit 21 may be a device that performs wireless communication or may be a device that performs wired communication.

The storage unit 22 is made up of a storage device such as a magnetic hard disk device or a semiconductor storage device. The storage unit 22 stores data to be used by the control unit 23. The storage unit 22 stores, for example, user gene information and medicine-related information. The storage unit 22 may further store feedback information.

The user gene information includes gene information for each user. The gene information for each user may be acquired by the user having blood taken in a hospital or the like and the taken blood being analyzed. For example, an analysis for detecting a difference in a type of a DNA sequence that is different in only one base, which is mainly called SNP (single nucleotide polymorphism), via, e.g., a microarray may be performed. Such analysis may be performed for an entire genome of the user or may be performed for a genome of a particular region of the user. Such analysis may be performed by, for example, a genotyping service provider. The user gene information includes information relating to gene polymorphisms of each user. The user gene information may include information relating to genotypes (sets of genes) and expressed genes. The user gene information may be registered in association with user IDs assigned to the respective users. The user IDs used in the user gene information management device 20 and user IDs used in the information provision server 30 may be different from each other. In this case, in the storage unit 22, a table indicating an association between respective user IDs assigned to a same user may be registered. Also, respective user IDs assigned to a same user may be associated with each other by using, for example, other identification information assigned to the user (for example, the number of a certification of insurance).

As described above, the medicine-related information includes the caution medicine information and the gene-related information. Fig. 5 is a diagram illustrating a specific example of the caution medicine information. The caution medicine information includes information indicating medicines requiring caution in excess of a predetermined level in handling, for each user. The "user ID" in Fig. 5 is identification information uniquely indicating each user. For example, the example in Fig. 5 indicates that a user with user ID "A1" needs caution for phenytoin and warfarin.

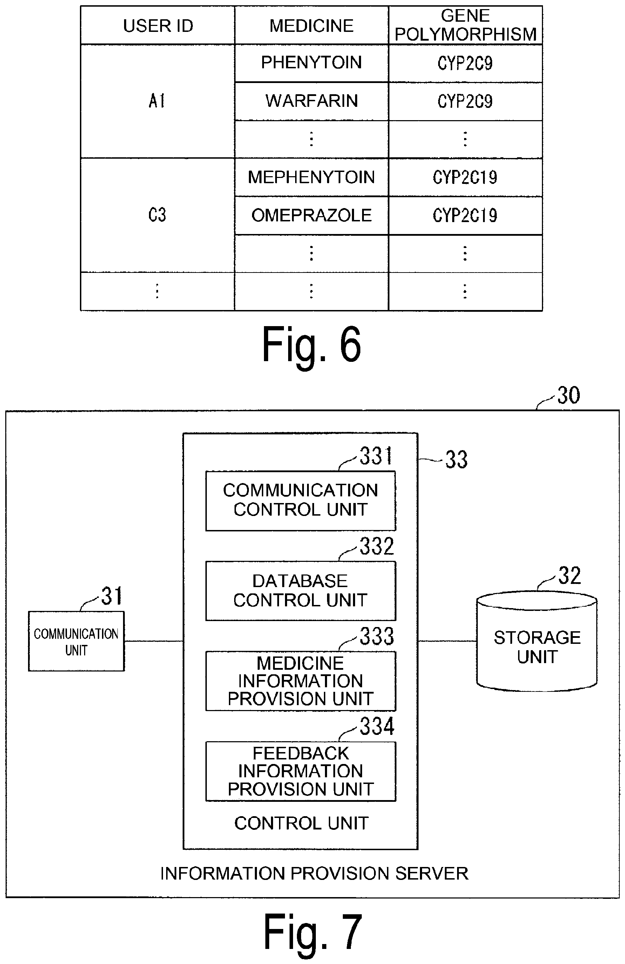

Fig. 6 is a diagram illustrating a specific example of the gene-related information. The gene-related information includes information indicating medicines requiring caution in excess of a predetermined level in handling, for each user and information indicating gene polymorphisms that cause the medicines to require caution. The "user ID" in Fig. 6 is identification information uniquely indicating each user. For example, the example in Fig. 6 indicates that the user with user ID "A1" needs caution for phenytoin, which is attributable to having a variant involved in metabolic activity for phenytoin among gene polymorphisms of gene CYP2C9.

The control unit 23 is made up of a processor such as a CPU, and a memory. The control unit 23 functions as a communication control unit 231, a database control unit 232 and an information provision unit 233 by the processor executing a program. Note that all or some of the functions of the control unit 23 may be implemented by using hardware such as an ASIC, a PLD or an FPGA. The above program may be recorded on a computer-readable recording medium. The computer-readable recording medium is, for example, a portable medium such as a flexible disk, a magneto-optical disk, a ROM, a CD-ROM or a semiconductor storage device (for example, an SSD) or a storage device, such as a hard disk or a semiconductor storage device, incorporated in a computer system. The above program may be transmitted via a telecommunication channel.

The communication control unit 231 controls communication with another device via the communication unit 21. The database control unit 232 updates the medicine-related information stored in the storage unit 22. For example, where new gene information of a user is obtained, the database control unit 232 records the obtained gene information in the storage unit 32. Upon the obtainment of the new gene information for the user, the database control unit 232 generates medicine-related information (caution medicine information and gene-related information) based on the obtained gene information and the medicine information recorded in the medicine information database 10. For example, it is possible that: the database control unit 232 checks whether or not the obtained new gene information of the user and gene polymorphism information pieces in the medicine information match each other, on a one-by-one basis; and if there is a gene polymorphism information piece matching the new gene information, medicine information and effect information associated with the gene polymorphism are acquired from the medicine information to generate medicine-related information. The database control unit 232 records the generated medicine-related information in the storage unit 22.

The information provision unit 233 provides caution medicine information to the information provision server 30, only for a user whose primary consent has been obtained in advance. Furthermore, for a user whose primary consent has been obtained in advance and whose secondary consent has subsequently been obtained, the information provision unit 233 provides relevant gene-related information to a predetermined other device. In this case, the permission condition is that the primary consent and the secondary consent have been obtained. The primary consent may be, for example, electronically obtained when a predetermined application is installed in the user terminal 40 or may be obtained via a predetermined written document. Once the primary consent is obtained, the primary consent may be maintained as being obtained until a request for cancellation of the primary consent is explicitly made by the user. For each user, information indicating whether or not a primary consent has been obtained may be recorded in the storage unit 22.

The secondary consent may be required each time a request for provision of gene-related information is made by the user. In other words, even though the secondary consent has been obtained once, if a request for provision of gene-related information is made again, the information provision unit 233 may ask for the user's secondary consent for such request. Such configuration enables proper protection of information relating to the user's genes.

Fig. 7 is a schematic diagram illustrating an example configuration of the information provision server 30. The information provision server 30 is made up of am information processing device such as a personal computer or a server device. The information provision server 30 does not need to be managed by an organization asked for analysis of gene information by a user or an organization allowed to manage gene information. The information provision server 30 may be a device managed by an organization that performs a service for providing information relating to medicines prescribed for users. The information provision server 30 includes a communication unit 31, a storage unit 32 and a control unit 33.

The communication unit 31 is a communication device. The communication unit 31 may be configured as, for example, a network interface. The communication unit 31 performs data communication with another device via the network 90 according to control by the control unit 33. The communication unit 31 may be a device that performs wireless communication or may be a device that performs wired communication.

The storage unit 32 is made up of a storage device such as a magnetic hard disk device or a semiconductor storage device. The storage unit 32 stores data to be used by the control unit 33. The storage unit 32 stores, for example, caution medicine information provided by the user gene information management device 20. The storage unit 32 may store, for example, information relating to medicines prescribed in the past (medicine prescription history information: for example, information recorded on a medicine notebook), for each user. Such information relating to medicines includes, for example, medicine types, product names, quantities and methods of use.

The control unit 33 is made up of a processor such as a CPU and a memory. The control unit 33 functions as a communication control unit 331, a database control unit 332, a medicine information provision unit 333 and a feedback information provision unit 334 by the processor executing a program. Note that all or some of the functions of the control unit 33 may be implemented by using hardware such as an ASIC, a PLD or an FPGA. The above program may be recorded on a computer-readable recording medium. The computer-readable recording medium is, for example, a portable medium such as a flexible disk, a magneto-optical disk, a ROM, a CD-ROM or a semiconductor storage device (for example, an SSD (solid-state drive)) or a storage device, such as a hard disk or a semiconductor storage device, incorporated in a computer system. The above program may be transmitted via a telecommunication channel.

The communication control unit 331 controls communication with another device via the communication unit 31. The database control unit 332 updates the caution medicine information stored in the storage unit 32. For example, it is possible that: the database control unit 332 inquires of the user gene information management device 20 about whether or not there is information for update, at a predetermined timing and if there is information for update, acquires the information for update for the caution medicine information and stores the information for update in the storage unit 32.

The medicine information provision unit 333 manages medicine prescription history information recorded for each user. Upon acquisition of information relating to newly prescribed medicines from the user terminal 40, the medicine information provision unit 333 registers the acquired information in the medicine prescription history information in the storage unit 32. The medicine information provision unit 333 determines whether or not a medicine included in caution medicine information for the relevant user is included in the newly prescribed medicines. If a medicine included in the caution medicine information is prescribed, the medicine information provision unit 333 transmits information indicating a warning (alert) for the medicine to the user terminal 40 and/or the dispensing terminal 50. Upon reception of a prescription history request including a user ID from the user terminal 40, the medicine information provision unit 333 reads prescription history information registered in the storage unit 32 in association with the received user ID and transmits the prescription history information to the user terminal 40.

Upon acquisition of feedback information, the feedback information provision unit 334 provides the acquired feedback information to another device. The feedback information provision unit 334 may provide the feedback information to, for example, the user gene information management device 20. The feedback information is information provided by a user who used a prescribed medicine. Feedback information may be acquired for estimation of an interaction between a plurality of medicines used by a user. Feedback information is information relating to, e.g., a feeling a user had after use of a medicine and a change in physical condition. The feedback information may be quantitative information (for example, information that can be acquired by a sensor, such as a heart rate, ECG (electrocardiogram) or a pulse rate, or a change in a predetermined component of blood). The feedback information may be any information as long as such information indicates a change in the body of the user, the change occurring due to use of the medicine. The feedback information may further include the following information: information indicating a medicine used and a timing (for example, a date and time) of the use of the medicine; time passed from the use of the medicine until a change in physical condition of the user; a length of time during which the change occurred in the physical condition of the user (duration of the effect); information indicating whether or not there was a side effect; information indicating whether or not there is a tendency for recovery from a symptom; information indicating foods and drinks the user had within a predetermined period of time before and after intake of the medicine; and information indicating foods and drinks the user ingested and a timing (for example, a date and time) of the ingestion. For example, acquisition of information indicating foods and drinks ingested by a user enables estimation of an interaction between a medicine used by the user and the foods and drinks ingested by the user. The feedback information may be, for example, input to the user terminal 40 by the user and transmitted to the information provision server 30 from the user terminal 40 via the network 90. Respective values in the feedback information may directly be input to the user terminal 40 by the user or by being selected by the user from choices defined in advance. The feedback information provision unit 334 may record the acquired feedback information in the storage unit 32.

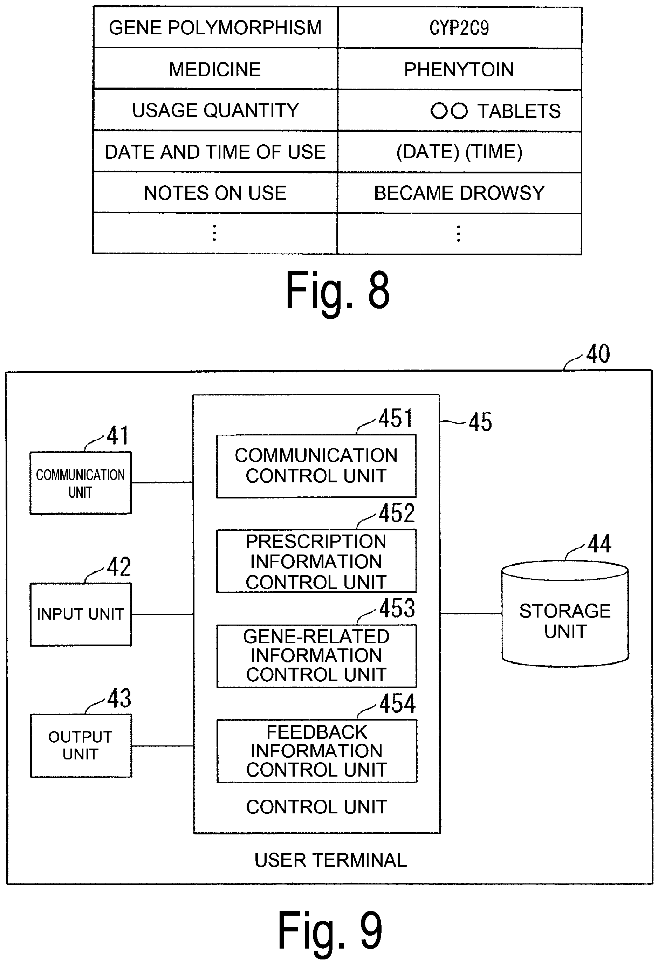

Fig. 8 is a diagram illustrating a specific example of the feedback information. The feedback information includes, for example, a related gene polymorphism of the user who provides the feedback, a medicine used, a usage quantity, a date and time of use, the user's feelings on the use (notes on use).

Here, one of purposes for acquiring the feedback information will be described. Side effects, effects and efficacy of a medicine itself are accumulated through pharmaceutical companies' tests and know-how of doctors, pharmacists and the like. However, it is contemplated that only very few side effects, effects and efficacy occurring when a plurality of medicines are administered simultaneously or within a period of time in which the medicines can interact with each other are accumulated in comparison with those for the medicines themselves. It is all the more so with a case where the plurality of medicines are manufactured by different pharmaceutical companies. Therefore, accumulation and management of such feedback information enables accumulation of interactions of medicines for each genotype. The feedback information may be stored as interactions, with no genotype being taken into consideration.

Fig. 9 is a schematic diagram illustrating an example configuration of the user terminal 40. The user terminal 40 is made up of an information processing device such as a personal computer, a smartphone, a mobile phone, a handheld game machine or a wearable computer. The user terminal 40 includes a communication unit 41, an input unit 42, an output unit 43, a storage unit 44 and a control unit 45.

The communication unit 41 is a communication device. The communication unit 41 may be configured as, for example, a network interface. The communication unit 41 performs data communication with another device via the network 90 according to control by the control unit 45. The communication unit 41 may be a device that performs wireless communication or may be a device that performs wired communication.

The input unit 42 is made up of an existing input device such as a keyboard, a pointing device (e.g., a mouse or a tablet), a button and/or a touch panel. The input unit 42 is operated by the user when the user's instruction is input to the user terminal 40. The input unit 42 may be an interface for connecting the input device to the user terminal 40. In this case, the input unit 42 inputs an input signal generated according to an input of the user in the input device, to the user terminal 40. The input unit 42 may be made up of a microphone and a speech recognition device. In this case, the input unit 42 recognizes words said by the user and inputs character string information that is a result of the recognition to the user terminal 40. The input unit 42 may be configured in any manner as long as such configuration allows input of the user's instruction to the user terminal 40.

The output unit 43 is made up of an output device and outputs data to the user of the user terminal 40. The output device may be made up of, for example, a device that outputs images and characters to a screen. For example, the output device can be made up of, e.g., a CRT (cathode ray tube), a liquid-crystal display or an organic EL (electro-luminescent) display. Also, the output device may be made up of a device that converts text into speech and outputs the speech. In this case, the output device can be made up of a speech synthesis device and an audio output device (speaker). The output unit 43 may be an interface for connecting the output device to the user terminal 40.

The storage unit 44 is made up of a storage device such as a magnetic hard disk device or a semiconductor storage device. The storage unit 44 stores data to be used by the control unit 45. The storage unit 44 may store, for example, data of a predetermined application installed in the user terminal 40.

The control unit 45 is made up of a processor such as a CPU and a memory. The control unit 45 functions as a communication control unit 451, a prescription information control unit 452, a gene-related information control unit 453 and a feedback information control unit 454 by the processor executing a program. Note that all or some of the functions of the control unit 45 may be implemented by using hardware such as an ASIC, a PLD or an FPGA. The above program may be recorded on a computer-readable recording medium. The computer-readable recording medium is, for example, a portable medium such as a flexible disk, a magneto-optical disk, a ROM, a CD-ROM or a semiconductor storage device (for example, an SSD) or a storage device, such as a hard disk or a semiconductor storage device, incorporated in a computer system. The above program may be transmitted via a telecommunication channel.

The communication control unit 451 controls communication with another device via the communication unit 41.

The prescription information control unit 452 manages information relating to a medicine newly prescribed for the user and information relating to medicines prescribed in the past. Processing for acquiring information relating to a medicine newly prescribed for the user and processing for acquiring information relating to medicines prescribed for the user in the past will be described below, respectively.

The prescription information control unit 452 acquires, for example, information (e.g., a type, a product name, a quantity and a method of use) relating a medicine newly prescribed for the user and transmits the information to the information provision server 30 together with the user ID. The information relating to the prescribed medicine may be input by, for example, the user operating the input unit 42 or may be input by encoded information such as a bar code printed on the prescription or the like being acquired. In this case, the encoded information such as a bar code may be information resulting from the information relating to the prescribed medicine itself being encoded or may be information resulting from information (for example, a URL) for accessing, e.g., a server that storages the information relating to the prescribed medicine to acquire the information being encoded. The encoded information such as a bar code is acquired via, for example, a device such as a bar code reader or an image pickup device included in the user terminal 40. Based on the encoded information such as a bar code, the prescription information control unit 452 acquires the information relating to the medicine newly prescribed for the user.

The prescription information control unit 452 acquires information relating to medicines prescribed for the user in the past (medicine prescription history information) by accessing the information provision server 30. For example, the prescription information control unit 452 transmits a prescription history request including the user ID to the information provision server 30. Upon reception of the prescription history request, the information provision server 30 transmits information indicating a prescription history for a predetermined period of time, the prescription history being registered in association with the user ID, to the user terminal 40. The prescription information control unit 452 outputs the acquired information via the output unit 43.

The gene-related information control unit 453 requests gene-related information from the user gene information management device 20 according to the user's operation. Upon reception of the gene-related information from the user gene information management device 20, the gene-related information control unit 453 outputs the received gene-related information from the output unit 43.

The feedback information control unit 454 requests an input of feedback information from the user. Such request may be made by, for example, predetermined information being output via the output unit 43. For example, the request for an input of feedback information may be made by a screen that enables an input of feedback information being displayed on an image display device (output unit 43). For example, the request for an input of feedback information may be made by a screen that enables an input of feedback information being displayed on an image display device (output unit 43) and a speech that urges an input of feedback information being output from a speaker (output unit 43). The request may be made in any manner. Upon feedback information being input by the user, the feedback information control unit 454 transmits the input feedback information to another device.

If no information relating to the user's gene is included in the feedback information, the feedback information may be transmitted to the information provision server 30. In this case, the feedback information may be transmitted together with the user ID indicating the user. A device that acquires the feedback information through the information provision server 30 (for example, the user gene information management device 20) may record gene information of the user and the feedback information in association with each other based on the user ID. As a result of such processing being performed, the gene information and the feedback information can be managed in a linked manner in the user gene information management device 20 while the gene information, which is private information, is kept secret from the information provision server 30.

If information relating to the user's gene is included in the feedback information, the feedback information may be transmitted to a particular device that manages gene information such as the user gene information management device 20 without being transmitted to the information provision server 30.

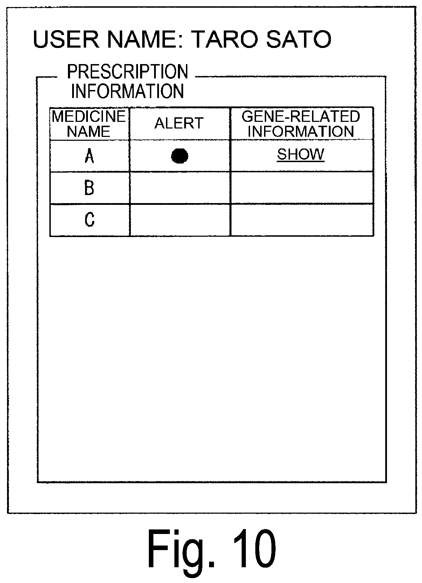

Figs. 10 to 18 are diagrams each illustrating an example output of the output unit 43 of the user terminal 40.

Fig. 10 is a diagram illustrating an example of an output mode of prescription information. As illustrated in Fig. 10, based on acquired information relating to a prescription, the medicine information provision unit 333 of the user terminal 40 may display names of prescribed medicines (medicine names) and information relating to whether or not there is an alert for any of the medicines, and if there is such alert, an operation object for outputting information related to a gene that is a basis for the alert, on a display. The operation object being operated indicates obtainment of a secondary consent from the user. In Fig. 10, the operation object is a character string "SHOW". This character string is provided with a hyperlink and a request for gene-related information may be transmitted to a predetermined destination of the hyperlink in response to operation of the hyperlink by the user. In this case, it is desirable that the user gene information management device 20 be set as the destination of the hyperlink.

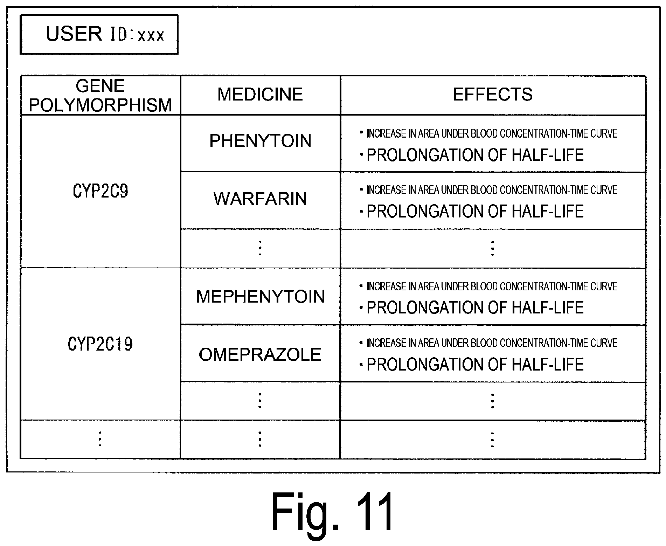

Upon the user clicking the character string "SHOW", the user terminal 40 transmits a request including the user ID and a name or an identifier of the medicine for which the alert has been issued, to the user gene information management device 20. Upon reception of the request, the user gene information management device 20 transmits a response including the gene-related information. The response may include information for displaying a user interface of the user gene information management device 20 on a browser. Fig. 11 is a diagram illustrating a specific example of the user interface displayed on the browser of the user terminal 40. As illustrated in Fig. 11, as a result of the browser of the user terminal 40 functioning as the user interface of the user gene information management device 20, the user's subsequent operation can be processed on the user gene information management device 20. On the user interface displayed on the browser of the user terminal 40, for example, respective values indicating related gene polymorphisms, medicines and effects may be displayed in association with one another. Such display may be, for example, a display indicating the table of medicine information illustrated in Fig. 3.

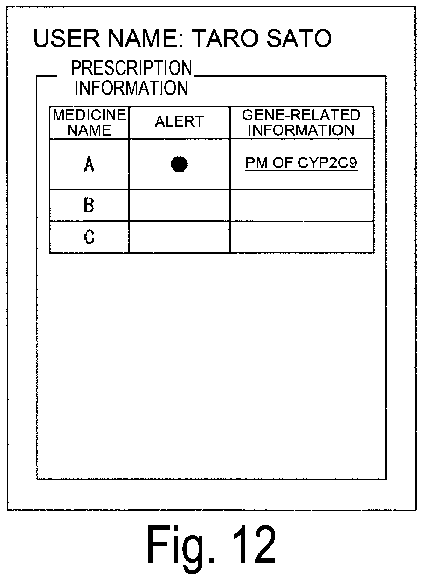

Fig. 12 is a diagram illustrating another example of an output mode in which gene-related information is displayed. In Fig. 12, another example screen after the character string "SHOW" being operated in Fig. 10 is illustrated. In the example in Fig. 12, gene-related information is displayed at the position in which the character string "SHOW" (operation object) had been displayed.

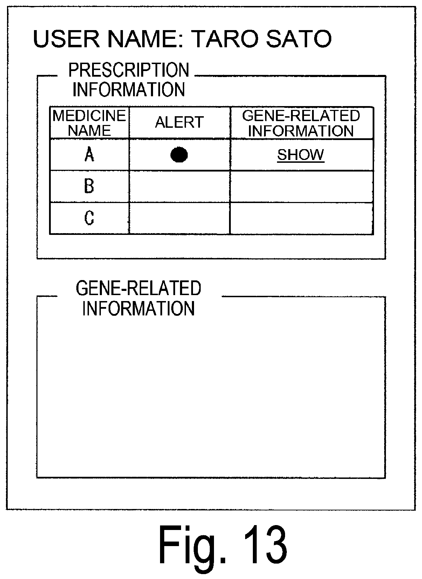

Fig. 13 is a diagram illustrating an example of an output mode of prescription information. In Fig. 13, a display pane for displaying gene-related information is displayed in addition to the prescription information illustrated in Fig. 10.

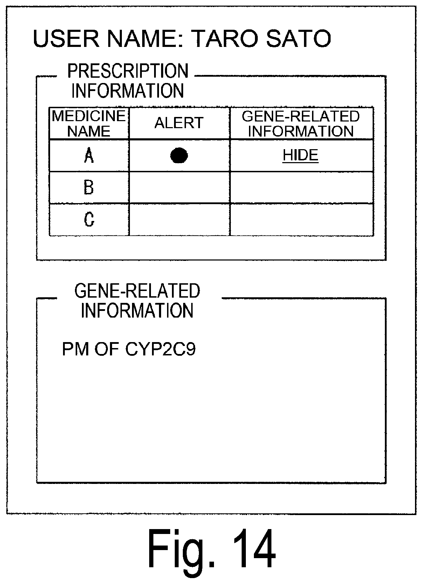

Fig. 14 is a diagram illustrating an example of an output mode in which gene-related information is displayed. In Fig. 14, an example screen after the character string "SHOW" being operated in Fig. 13 is illustrated. Gene-related information is displayed not at the position at which the character string "SHOW" (operation object) had been displayed but in the gene-related information display pane provided in advance. Also, in Fig. 14, at the position at which the character string "SHOW" (operation object) is displayed in Fig. 13, an operation object that is a character string "HIDE" is displayed. If this operation object is operated, the gene-related information control unit 453 performs control to hide the gene-related information displayed in the gene-related information display pane. For example, control to make a transition back to the screen in Fig. 13 may be performed.

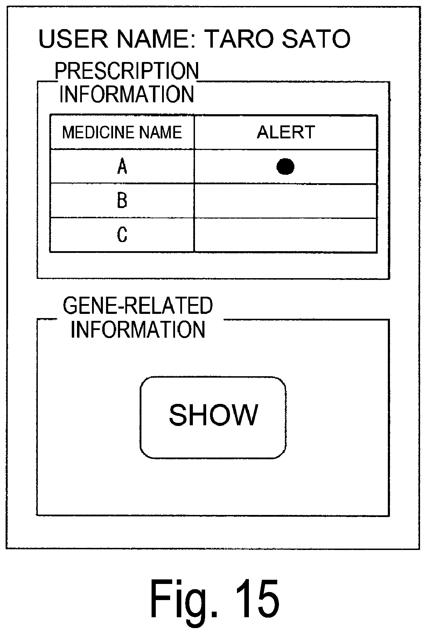

Fig. 15 is a diagram illustrating an example of an output mode of prescription information. As illustrated in Fig. 15, based on acquired information relating to a prescription, the medicine information provision unit 333 of the user terminal 40 may display names of prescribed medicines (medicine names) and information indicating whether or not there is an alert for any of the medicines in a prescription information display pane. Also, if an alert is issued, an operation object for outputting information related to a gene that is a basis for the alert may be displayed in the gene-related information display pane provided in advance. In the example in Fig. 15, the operation object is a button including a character string "SHOW". In response to this button being operated by the user, the gene-related information control unit 453 requests transmission of gene-related information from the user gene information management device 20.

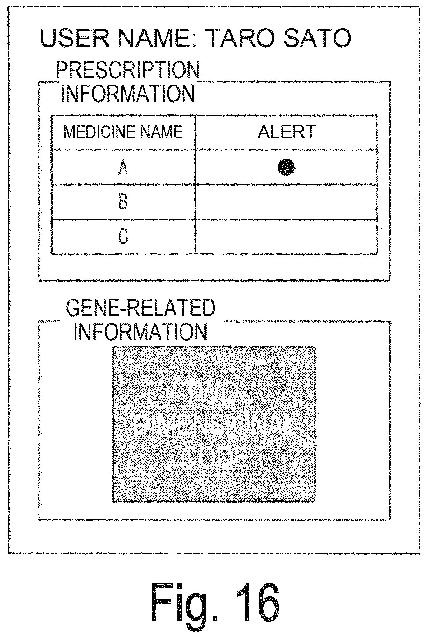

Fig. 16 is a diagram illustrating an example of an output mode in which gene-related information is displayed. In Fig. 16, an example screen after the button "SHOW" being operated in Fig. 15 is illustrated. At the position at which the button "SHOW" (operation object) has been displayed, encoded information of gene-related information is displayed as gene-related information. In Fig. 16, as a specific example of the encoded information, a two-dimensional code is displayed. For example, upon this two-dimensional code being decoded as a result of the dispensing terminal 50 used in a dispensing facility 501 being operated, the gene information of the user is output in the dispensing terminal 50. If the encoded information is information obtained by encoding the gene-related information itself, the dispensing terminal 50 can promptly display the gene information on a display by decoding the encoded information. The encoded information may be information resulting from information (for example, a URL) for accessing, e.g., a server that accumulates gene-related information (for example, the user gene information management device 20) to acquire the information being encoded. In this case, the dispensing terminal 50 may acquire the gene information of the user by accessing such other device based on the information obtained by the decoding, and display the gene information.

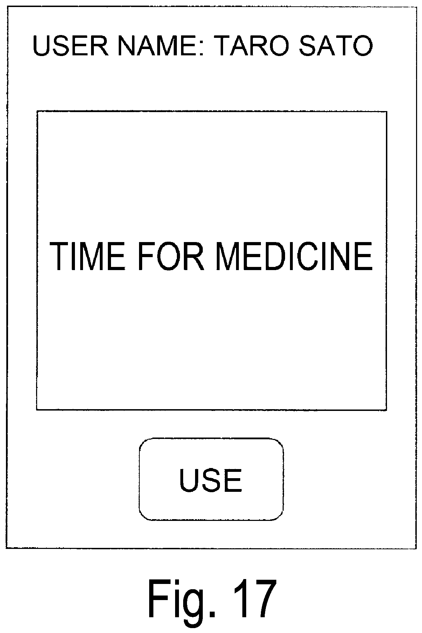

Fig. 17 is a diagram illustrating an example screen of a use reminder. The prescription information control unit 452 determines a timing for the user to use a medicine based on information relating to currently prescribed medicines (in particular, a method of use). For example, taking the medicine at the morning and evening after a meal is defined as a method of use, the prescription information control unit 452 displays a screen of a use reminder at each of timings a predetermined period of time from a breakfast time and a dinner time set in advance as defaults or set for the user. As illustrated in Fig. 17, characters and an image indicating that it is a timing for using the medicine (for example, a character string "TIME FOR MEDICINE") may be displayed on the screen of the use reminder. Furthermore, as a button for indicating the user's use to the user terminal 40, an operation object may be displayed. In the example in Fig. 17, a button including characters "USE" is displayed as an operation object. Upon the user operating the operation object, the prescription information control unit 452 records the use of the medicine at that timing by the user.

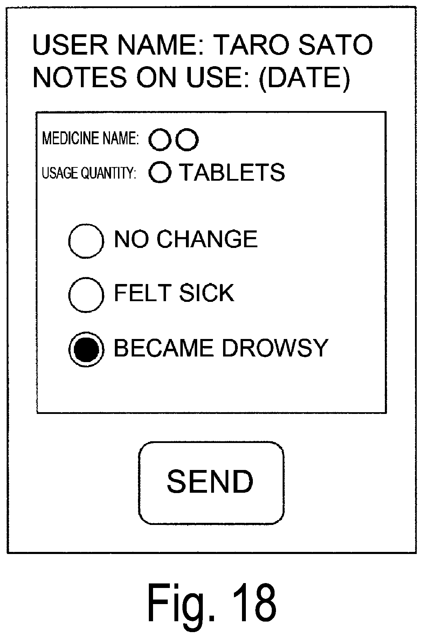

The feedback information control unit 454 may display a questionnaire screen (feedback information input screen) after the screen of the use reminder being displayed. Fig. 18 is a diagram illustrating a specific example of the questionnaire screen. The questionnaire screen includes at least characters and an image that urge an input of predetermined feedback information. In the example in Fig. 18, three choices for a condition of the user after the use are displayed as feedback information. Each choice may be displayed in association with, e.g., an image for an input such as a checkbox. The user inputs feedback information on the questionnaire screen. The feedback information control unit 454 acquires the feedback information input by the user, via the questionnaire screen. Note that in Fig. 18, information indicating the medicine used by the user (medicine name) and information indicating a quantity of the medicine used (usage quantity) are displayed on the screen in advance. However, these information pieces may be input according to the user's operation. Also, although in Fig. 18, a date and time of use of the medicine by the user is indicated as notes on use, such date and time may directly be input by the user, may be acquired by the feedback information control unit 454 as a date and time of display of the screen in Fig. 18 by the user or may be acquired as a date and time of operation of the button "USE" in Fig. 17.

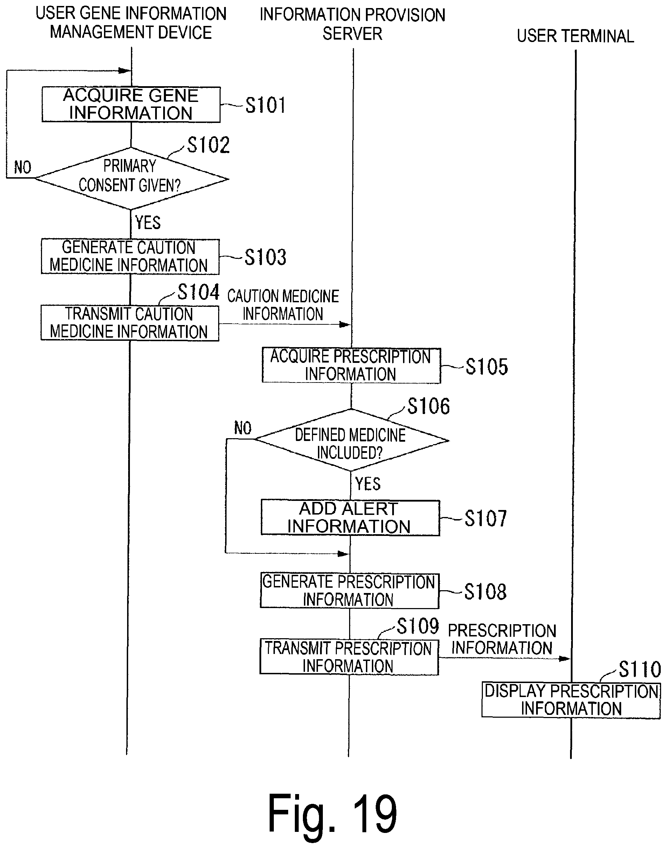

Fig. 19 is a sequence chart illustrating a specific example of operation of the information provision system 100. Upon acquisition of new gene information of the user (step S101), the user gene information management device 20 determines whether or not a primary consent has been obtained from the user that is a subject of the gene information has been acquired. If the primary consent has not been obtained (step S102: NO), the processing in the user gene information management device 20 ends and subsequently, processing in step S105 onwards is performed by the information provision server 30 and the user terminal 40.

On the other hand, if the primary consent has been obtained (step S102: YES), the user gene information management device 20 generates caution medicine information for the subject user (step S103). Then, the user gene information management device 20 transmits the caution medicine information for the subject user to the information provision server 30 (step S104).

Upon reception of the caution medicine information, the information provision server 30 records the caution medicine information in the storage unit 32. Subsequently, upon acquisition of prescription information (step S105), the information provision server 30 determines whether or not a medicine defined in the caution medicine information is included in medicines included in the prescription information (step S106). If a medicine defined in the caution medicine information is included in medicines included in the prescription information (step S106: YES), the information provision server 30 generates prescription information with alert information added to the medicine (steps S107 and S108). On the other hand, if no medicine defined in the caution medicine information is included in the medicines included in the prescription information (step S106: NO), the information provision server 30 generates prescription information with no alert information added (step S108). Subsequently, the information provision server 30 transmits the generated prescription information to the user terminal 40 (step S109).

Upon reception of the prescription information from the information provision server 30, the user terminal 40 outputs the received prescription information. For example, the user terminal 40 displays the prescription information on the display, which is a mode of the output unit 43 (step S110).

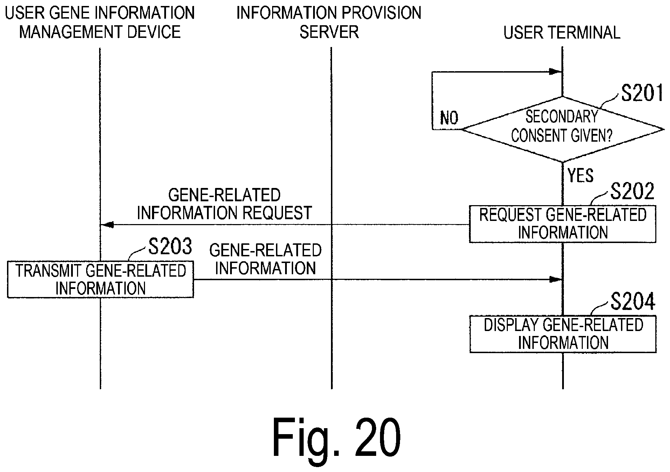

Fig. 20 is a sequence chart illustrating a specific example of a flow of operation of the information provision system 100. Fig. 20 specifically illustrates a flow of processing subsequent to step S110 in Fig. 19. In the user terminal 40, the prescription information is displayed until an operation to indicate a secondary consent is performed (step S201: NO). Note that, although not illustrated, the display of the prescription information may be terminated if an operation to terminate the display of the prescription information is performed. Upon an operation to indicate a secondary consent being performed in the user terminal 40 (step S201: YES), the user terminal 40 requests gene-related information from the user gene information management device 20.

Upon reception of the gene-related information request indicating that the secondary consent has been given from the user terminal 40, the user gene information management device 20 generates gene-related information for the user. Then, the user gene information management device 20 transmits the generated gene-related information to the user terminal 40 (step S203). Upon reception of the gene-related information, the user terminal 40 outputs the received gene-related information. For example, the user terminal 40 displays the gene-related information on the display, which is a mode of the output unit 43 (step S204).

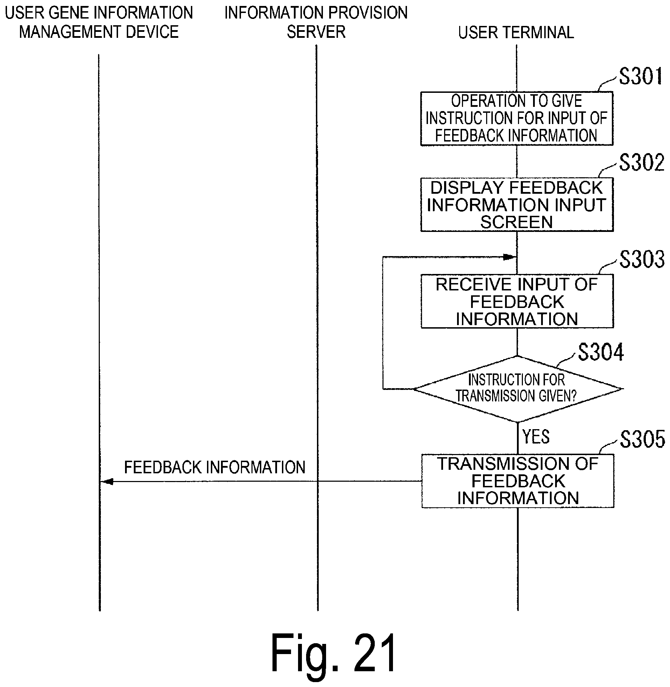

Fig. 21 is a sequence chart illustrating a specific example of a flow of operation of the information provision system 100. Fig. 21 specifically illustrates a flow of processing relating to feedback information. Upon an operation indicating an input of feedback information being performed in the user terminal 40 (step S301), the feedback information provision unit 454 displays a feedback information input screen in the user terminal 40 (step 302). Upon a value being input to each of input fields provided in the feedback information input screen, the feedback information control unit 454 receives the input values. The values may be directly input, may be input by selection from choices set in advance or may be input in another manner. Subsequently, upon an instruction to transmit feedback information being provided by the user (step S304: YES), the feedback information control unit 454 generates feedback information including the information input by the user until that time and transmits the feedback information to a predetermined other device (step S305). In the example in Fig. 21, the feedback information control unit 454 transmits the feedback information to the user gene information management device 20.

The information provision system 100 configured as above enables proper dispending according to individual differences relating to medicine. A more specific description will be provided as follows. In the information provision system 100, medicines requiring caution are determined for each individual based on gene information of the individual and provided to the information provision server 30 as caution medicine information. In the information provision server 30, whether or not a medicine requiring caution for the relevant individual is included in medicines included in a new prescription issued to the user is determined. Then, if such medicine is included, an alert for the medicine is issued to the user. Therefore, for a medicine not requiring caution for a general person but requiring caution because of an individual difference based on a gene, the user can consult with a pharmacist or a doctor about, e.g., a method of use for the medicine with caution.

Also, in the information provision system 100, caution medicine information provided to the information provision server 30 includes no information relating to individuals' genes. Therefore, it is possible to keep gene information, which is private information, secret while the aforementioned effect being exerted.

Also, in the information provision system 100, if the user wishes that dispensing be performed based on more detailed gene information, gene information (gene-related information), which is private information, can be acquired by an operation for secondary consent being performed. In this case, the user can consult with a pharmacist or a doctor about, e.g., prescription or a method of use with more specific caution based on his/her genes. Also, in the above-described processing, gene information can be acquired from the user gene information management device 20 without intervention of the information provision server 30. Therefore, it is possible to keep gene information, which is private information, secret while the aforementioned effect being exerted.

Also, in the information provision system 100, the user who used a prescribed medicine can input information relating to, e.g., a feeling the user had after the use of the medicine and a change in physical condition to the user terminal 40 as feedback information. The feedback information is provided to another device such as the user gene information management device 20. Therefore, it is possible to develop better medicines based on, e.g., changes in physical conditions of users who actually used medicines. In view of such purpose, it is more preferable that gene information of the user be included in the feedback information. Note that if the user uses a plurality of medicines, it is possible to, for each medicine, give feedback on the medicine used, a quantity of the medicine used and a date and time of the use. Also, it is possible to give feedback on the user's subjective symptom and a date and time of the subjective symptom being exhibited. Where a plurality of medicines are used, it is difficult for the user to determine whether a side effect is caused by any of the medicines or a combination of the medicines; however, an analysis by an expert or the like is enabled by acquiring dates and times of use of the respective medicines and a date and time of a subjective symptom being exhibited.

### (Alterations)

The functions implemented by any of the devices may be implemented by a plurality of devices. For example, the user gene information management device may be implemented by a plurality of information processing devices. For example, the user gene information management device 20 may be implemented by being divided into an information processing device that performs the processing in the database control unit 232 and an information processing device that performs the processing in the information provision unit 233.

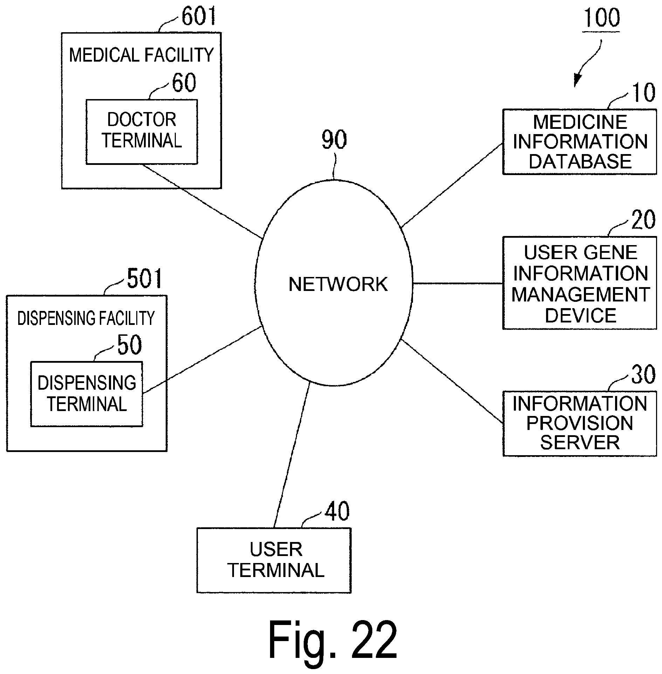

Fig. 22 is a schematic block diagram illustrating a system configuration of an alteration of the information provision system 100 according to the present invention. In the alteration of the information provision system 100, a doctor terminal 60 is included in an information provision system 100. The doctor terminal 60 is installed in a medical facility 601. The doctor terminal 60 is an information processing device that is used by a doctor who prescribes a medicine for a user. The doctor inputs a user ID of a user who is a patient to the doctor terminal 60. The user ID may be, for example, a user ID used in the medical facility 601. In this case, it is preferable that information associating the user ID used in the medical facility 601 and a user ID used in an information provision server 30 is registered in any device such as the information provision server 30. The doctor inputs information relating to a medicine supposed to be prescribed for the user who is a patient (hereinafter referred to as "prescription candidate information") to the doctor terminal 60. If a medicine included in caution medicine information for the user indicated by the input user ID is included in the input prescription candidate information, the doctor terminal 60 outputs information of that medicine. Note that as the information relating to a medicine, a name of the medicine itself may be used alone.

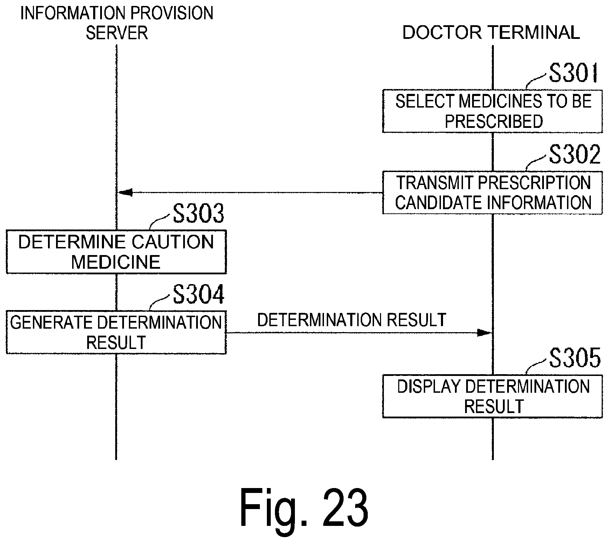

Fig. 23 is a sequence chart illustrating a specific example of a flow of operation of the alteration of the information provision system 100. The doctor inputs a user ID of a user who is a patient and prescription candidate information to the doctor terminal 60 (step S301). The doctor terminal 60 transmits the input prescription candidate information to the information provision server 30 (step S302).

The information provision server 30 determines whether or not a medicine defined in caution medicine information is included in medicines included in the prescription candidate information (step S303). If a medicine defined in the caution medicine information is included in the medicines included in the prescription candidate information, the information provision server 30 generates information indicating that the medicine requires caution (alert information), as a result of the determination. On the other hand, if no medicine defined in the caution medicine information is included in the medicines included in the prescription candidate information, the information provision server 30 generates information indicating such effect, as a result of the determination. Subsequently, the information provision server 30 transmits the generated determination result to the doctor terminal 60 (step S304).

Upon reception of the determination result from the information provision server 30, the doctor terminal 60 outputs the received determination result. For example, the doctor terminal 60 displays the determination result on a display (step S305).

As another alteration, for each medicine, an information provision server 30 may store a list indicating other medicines having a medicinal effect of a type that is the same as that of the medicine. For example, for each medicine, a list indicating other medicines that are usable as an alternative medicine for the medicine may be stored. If a medicine in caution medicine information for a relevant user is included in newly prescribed medicines or medicines in prescription candidate information, a medicine information provision unit 333 of the information provision server 30 searches for another medicine that is a medicine not included in the caution medicine information for the user and that is usable as an alternative medicine for that medicine. Then, the medicine information provision unit 333 of the information provision server 30 may transmit information indicating a result of the search (information indicating the other medicine searched for) to another device (a user terminal 40, a dispensing terminal 50 or a doctor terminal 60).

Although an embodiment of this invention has been described in detail above with reference to the drawings, a specific configuration of this invention is not limited to the embodiment but includes designs and the like without departing from the spirit of the invention.

### Industrial Applicability

The present invention is applicable to techniques for dispensing of medicine.

### Reference Signs List

100 Information provision system
10 Medicine information database
20 User gene information management device
30 Information provision server
40 User terminal
50 Dispensing terminal
90 Network
11 Communication unit
12 Storage unit
13 Control unit
131 Communication control unit
132 Database control unit
21 Communication unit
22 Storage unit
23 Control unit
231 Communication control unit
232 Database control unit
233 Information provision unit
31 Communication unit
32 Storage unit
33 Control unit
331 Communication control unit
332 Database control unit
333 Medicine information provision unit
334 Feedback information provision unit
41 Communication unit
42 Input unit
43 Output unit
44 Storage unit
45 Control unit
451 Communication control unit
452 Prescription information control unit
453 Gene-related information control unit
454 Feedback information control unit

## Claims

1. An information providing apparatus comprising:
a communication unit that communicates with another device via a network; and
a control unit that, based on information indicating a medicine requiring caution in use for a user, the information being obtained based on information relating to a gene of the user, and information indicating a medicine prescribed for the user, determines a medicine requiring caution in use for the user, the medicine requiring caution in use being of the medicine prescribed for the user, and provides information indicating a result of the determination to the other device.

2. The information providing apparatus according to claim 1, wherein as the information relating a medicine requiring caution in use for the user, the control unit provides a name of the medicine alone.

3. The information providing apparatus according to claim 1 or 2, wherein the control unit further provides information indicating another medicine that is usable as an alternative medicine for the medicine in addition to the information relating a medicine requiring caution in use for the user.

4. An information managing apparatus comprising:
a communication unit that communicates with another device via a network;
a storage unit that stores user gene information that is information relating to a gene of a user and caution medicine information indicating a medicine requiring caution in use for the user, the caution medicine information being obtained based on the user gene information; and
a control unit that provides the caution medicine information to the other device.

5. The information managing apparatus according to claim 4, wherein the control unit further provides a part or an entirety of the user gene information to the other device subject to a predetermined condition indicating obtainment of consent from the user being met.

6. The information managing apparatus according to claim 4 or 5, wherein for each user, the control unit generates the caution medicine information based on the user gene information.

7. A user terminal comprising:
a communication unit that communicates with another device via a network;
an output unit that outputs information to a user; and
a control unit that acquires information relating to a medicine requiring caution in use for the user, which is of the medicine prescribed for the user, and outputs the information relating to a medicine requiring caution in use to the user via the output unit.

8. The user terminal according to claim 7, further comprising an input unit that receives an input of information from a user,
wherein for the medicine prescribed for the user, the control unit acquires information indicating a condition of the user after use, via the input unit and provides the information to the other device via the communication unit.

9. An acquiring method comprising in order to estimate an interaction between a plurality of medicines used by a user:
a step of acquiring the medicines used by the user, an effect relating to a body of the user, the effect being exerted after use of the medicines by the user, and a time; and
a step of performing at least one of processing for transmitting the acquired medicines, effect and time to another device and processing for storing the acquired medicines, effect and time in a storage device.

10. The acquiring method according to claim 9, wherein the effect relating to the body of the user includes at least one of whether there is a side effect and a tendency for recovery from a symptom.

11. The acquiring method according to claim 9 or 10, wherein in order to further estimate an interaction between the medicines used by the user and foods and drinks ingested by the user, information indicating the food ingested by the user is further acquired.

12. A user interface comprising:
a medicine input field that receives an input of information indicating a medicine used by the user;
an effect input field that receives an input of information indicating an effect relating to a body of the user, the effect being exerted after use of the medicine by the user; and
a transmission determination field that receives an instruction for transmission of the information input by the user.

13. A database comprising:
a storage unit that, in order to estimate an interaction between a plurality of medicines used by a user, stores the medicines used by the user, an effect relating to a body of the user, the effect being exerted after use of the medicines by the user, and a time in association with one another.

14. An information providing method comprising:
a step of, based on information indicating a pharmaceutical formulation requiring caution in use/administration for a user, the information being obtained based on information relating to a gene of the user, and information indicating a pharmaceutical formulation prescribed for the user, determining a pharmaceutical formulation requiring caution in use/administration for the user, the pharmaceutical formulation requiring caution in use/administration being of the pharmaceutical formulation prescribed for the user; and
a step of providing information indicating a result of the determination to another device.

15. A control method comprising:
a step of acquiring information relating to a pharmaceutical formulation requiring caution in use/administration for a user, which is of the pharmaceutical formulation prescribed for the user; and
a step of outputting the information relating to a pharmaceutical formulation requiring caution in use/administration to the user.
